# EUROPÄISCHE PATENTANMELDUNG

(11) **EP 2 623 091 A2**
(43) Veröffentlichungstag der Anmeldung: **07.08.2013**
(21) Anmeldenummer: 12191939.3
(22) Anmeldetag: 09.11.2012
(51) Int. Cl.: A61K 8/41, A61K 8/73, A61Q 5/12, A61Q 5/00

(54) **Haarpflegemittel**

(30) Priorität: 07.12.2011 DE 102011087906
(71) Anmelder: Henkel AG&Co. KGAA, 40589 Düsseldorf (DE)
(72) Erfinder: Battermann, Marlene, 21271 Asendorf (DE); Scheunemann, Volker, 21339 Lüneburg (DE); Hippe, Thomas, 25482 Appen (DE)

(57) **Zusammenfassung**

Die Erfindung betrifft Mittel zur Konditionierung von Keratinfasern, insbesondere von Haaren, die in einem kosmetischen Träger
a) mindestens eine Verbindung der nachfolgenden Formel (I) 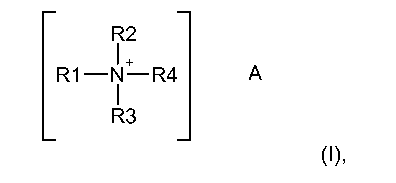 in der höchstens drei Reste R1 bis R4 unanhängig voneinander für eine gesättigte oder ungesättigte, verzweigte oder unverzweigte Alkylgruppe mit 1 bis 4 C-Atomen stehen, mindestens ein Rest R1 bis R4 für eine gesättigte oder ungesättigte, verzweigte oder unverzweigte Alkylkette mit 8 bis 30 C-Atomen steht, und A ein physiologisch verträgliches organisches oder anorganisches Anion bedeutet,
b) mindestens ein Esterquat und
c) mindestens ein quaternisiertes Hydroxyethylcellulosepolymer enthalten, das kationische Trimethylammonium- und Dimethyldodecylammoniumgruppen als Substituenten aufweist.

## Beschreibung

Die Erfindung liegt auf dem Gebiet der Kosmetik und betrifft Mittel zur Konditionierung von Keratinfasern, insbesondere von Haaren, die in einem kosmetischen Träger eine spezifische Mischung dreier quaternärer Ammoniumverbindungen enthalten.

Die kosmetische Behandlung keratinischer Fasern, insbesondere menschlicher Haare, ist ein wichtiger Bestandteil der menschlichen Körperpflege. So wird menschliches Haar heute in vielfältiger Weise mit haarkosmetischen Zubereitungen behandelt.

Vielfach wiederholte Haarbehandlungen, wie beispielsweise oxidative oder reduktive Haarbehandlungen, aber auch die allzu häufige Reinigung der Haare, die Einwirkung von Fönhitze sowie Umwelteinflüsse und/oder mechanische Einwirkungen, können Haare in ihrer Struktur schädigen.

Die Folgen der Schädigungen sind oftmals Haarbruch, Spliss sowie glanzlose, stumpfe und/oder fliegende Haare.

Unter "Schädigung" wird der Verlust von natürlichen Lipiden aus der Cortex und der Cuticula verstanden. Einmal entfernt, können die Lipide nicht mehr natürlich nachproduziert werden, und müssen der Kopfhaut und den Haaren in Form kosmetischer Mittel wieder zugeführt werden.

Daher ist es üblich, gereinigte Haare einer speziellen Nachbehandlung zu unterziehen, um ihnen Pflegestoffe zuzuführen, die ihnen wieder ein gesundes Aussehen verleihen, die die Haarstruktur kräftigen sowie die Kopfhaut pflegen bzw. vor dem Austrocknen schützen.

Bei der Nachbehandlung werden Haare meist mit Haarspülungen und/oder Haarkuren behandelt, die üblicherweise eine Mischung aus Fettstoffen und quaternären Ammoniumverbindungen enthalten.

Es hat sich jedoch gezeigt, dass die Qualität und/oder Wirksamkeit der Nachbehandlungsprodukte je nach Wahl der Pflegekomponenten stark variieren kann.

So konnte beispielsweise beobachtet werden, dass die Abscheidung der Pflegekomponenten auf den Haaren -je nach Kombination der Wirkstoffe in dem Haarpflegemittel - nicht immer optimal ist, wodurch die Pflegeleistung der Mittel unbefriedigend wird.

Der vorliegenden Erfindung lag die Aufgabe zugrunde, konditionierende Haarbehandlungsmittel auf Basis einer pflegenden Wirkstoffkombination zu entwickeln, welche bei Anwendung der Mittel nachhaltig auf den Haarfasern abgeschieden wird.

Durch die erhöhte Abscheidung der Pflegewirkstoffe sollen behandelte Haare besser gepflegt werden, insbesondere sollen behandelte Haare einen verbesserten Griff und ein optisch ansprechendes Erscheinungsbild aufweisen.

Weiterhin sollte die Struktur geschädigter Haare durch die Anwendung der konditionierenden Haarbehandlungsmittel wieder gestärkt werden (Restrukturierung), insbesondere sollten behandelte Haare wieder elastisch, glatt und glänzend erscheinen.

Es wurde gefunden, dass sich eine bestimmte Wirkstoffkombination, bestehend aus einer Mischung dreier quaternärer Ammoniumverbindungen, zur Lösung dieser Aufgaben eignet.

Gegenstand der vorliegenden Erfindung ist daher Mittel ein zur Konditionierung von Keratinfasern, insbesondere von Haaren, das in einem kosmetischen Träger
a) mindestens eine Verbindung der nachfolgenden Formel (I) 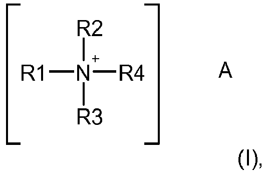 in der
   höchstens drei Reste R1 bis R4 unanhängig voneinander für eine gesättigte oder ungesättigte, verzweigte oder unverzweigte Alkylgruppe mit 1 bis 4 C-Atomen stehen, mindestens ein Rest R1 bis R4 für eine gesättigte oder ungesättigte, verzweigte oder unverzweigte Alkylkette mit 8 bis 30 C-Atomen steht, und A ein physiologisch verträgliches organisches oder anorganisches Anion bedeutet,
b) mindestens ein Esterquat und
c) mindestens ein quaternisiertes Hydroxyethylcellulosepolymer, welches kationische Trimethylammonium- und Dimethyldodecylammoniumgruppen als Substituenten aufweist, enthält.

Mittel zur Konditionierung von Keratinfasern, insbesondere von Haaren, im Sinne der vorliegenden Erfindung sind beispielsweise Haarspülungen, Haarkuren, Haarpackungen, Haar-Tonics, Haarspitzenfluids und/oder deren Kombinationen.

Unter "Restrukturierung" im Sinne der Erfindung ist eine Verringerung der durch verschiedenartigste Einflüsse entstandenen Schädigungen keratinischer Fasern zu verstehen. Hierbei spielt beispielsweise die Wiederherstellung der natürlichen Festigkeit eine wesentliche Rolle. Restrukturierte Fasern zeichnen sich durch einen verbesserten Glanz, durch einen verbesserten Griff und durch eine leichtere Kämmbarkeit aus. Zusätzlich weisen sie eine verbesserte Festigkeit und Elastizität auf. Ferner lässt sich eine erfolgreiche Restrukturierung physikalisch als Schmelzpunktserhöhung im Vergleich zur geschädigten Faser nachweisen. Je höher der Schmelzpunkt des Haares ist, desto fester ist die Struktur der Faser.

Als kosmetische Träger für die erfindungsgemäßen Mittel eignen sich besonders O/W - , W/O - und W/O/W - Emulsionen in Form von Cremes oder Gelen, aber auch tensidhaltige, schäumende Lösungen und/oder Dispersionen, Schaumaerosole oder andere Zubereitungen, die insbesondere für die Anwendung auf dem Haar geeignet sind. Die kosmetischen Träger können insbesondere wässrig oder wässrig-alkoholisch sein.

Ein geeigneter wässriger kosmetischer Träger enthält bevorzugt mindestens 50 Gew.-% Wasser. Unter wässrig-alkoholischen kosmetischen Trägern sind im Sinne der vorliegenden Erfindung bevorzugt wässrige Lösungen, enthaltend 0,5 bis 70 Gew.-% eines C₁-C₆-Alkohols, insbesondere Methanol, Ethanol bzw. Propanol, Isopropanol, Butanol, Isobutanol, tert.-Butanol, n-Pentanol, iso-Pentanole, n-Hexanol, iso-Hexanole, Glykol, Glycerin, 1,2-Pentandiol, 1,5-Pentandiol, 1,2-Hexandiol oder 1,6-Hexandiol zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Insbesondere bevorzugt sind wässrige Träger.

Erfindungsgemäß bevorzugte Mittel enthalten die Wirkstoffkombination a), b) und c) in den folgenden Mengen - bezogen auf das Gesamtgewicht des Mittels -
- 0,01 bis 10 Gew.-%, bevorzugt 0,05 bis 8 Gew.-%, mehr bevorzugt 0,1 bis 7 Gew.-%, besonders bevorzugt 0,5 bis 6 Gew.-% und insbesondere 1 bis 5 Gew.-% der Komponente a),
- 0,01 bis 10 Gew.-%, bevorzugt 0,05 bis 8 Gew.-%, mehr bevorzugt 0,1 bis 7 Gew.-%, besonders bevorzugt 0,5 bis 6 Gew.-% und insbesondere 1 bis 5 Gew.-% der Komponente b), und
- 0,01 bis 5 Gew.-%, bevorzugt 0,02 bis 4 Gew.-%, mehr bevorzugt 0,03 bis 3 Gew.-%, besonders bevorzugt 0,04 bis 2 Gew.-% und insbesondere 0,05 bis 1 Gew.-% der Komponente c).

In bevorzugten Verbindungen a) gemäß Formel (I) steht/stehen
- zwei oder drei Reste R1 bis R4 für eine Methyl- oder eine Ethylgruppe,
- ein oder zwei Rest(e) R1 bis R4 für eine gesättigte oder ungesättigte, verzweigte oder unverzweigte Alkylkette mit 14 bis 26 C-Atomen, und
- A für ein Halogenidion, ein Sulfation der allgemeinen Formel RSO₃⁻, worin R die Bedeutung von gesättigtem oder ungesättigtem Alkylresten mit 1 bis 4 Kohlenstoffatomen hat, oder für einen anionischen Rest einer organischen Säuren wie Maleinsäure, Fumarsäure, Oxalsäure, Weinsäure, Citronensäure, Milchsäure oder Essigsäure.

Mehr bevorzugt sind Verbindungen a) gemäß Formel (I), in der
- drei Reste R1 bis R4 für eine Methylgruppe stehen,
- ein Rest R1 bis R4 für eine Cetyl-, Palmityl-, Stearyl-, Arachidyl- oder eine Behenylgruppe steht, und
- A für ein Chlorid oder ein Methosulfation steht.

Besonders bevorzugt ist die mindestens eine Verbindung a) nach Formel (I) ausgewählt aus Cetyltrimethylammoniumchlorid, Cetyltrimethylammoniummethosulfat, Behentrimethylammoniumchlorid und/oder Behentrimethylammoniummethosulfat. Diese Verbindungen können in dem erfindungsgemäßen Mittel einzeln oder in ihrer Kombination eingesetzt werden, wobei die Gesamtmenge an Verbindungen a) in dem Mittel bevorzugt maximal 10 Gew.-% beträgt, und wobei sich die Mengenangabe auf das Gesamtgewicht des erfindungsgemäßen Mittels bezieht.

Insbesondere bevorzugt enthält ein erfindungsgemäßes Mittel als Verbindung a) Behentrimethylammoniumchlorid in einer bevorzugten Menge von 0,1 bis 10 Gew.-%, mehr bevorzugt von 0,5 bis 8 Gew.-%, besonders bevorzugt von 0,75 bis 6 Gew.-% und insbesondere von 1 bis 5 Gew.-%, wobei sich die Mengenangaben auf das Gesamtgewicht des erfindungsgemäßen Mittels beziehen.

Unter "Esterquats" im Sinne der vorliegenden Erfindung werden bevorzugt Verbindungen der nachfolgenden Formel (II) verstanden 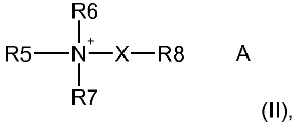 in der
die Reste R5, R6 und R7 jeweils unabhängig voneinander gleich oder verschieden sein können und die folgende Bedeutung haben:
- ein gesättigter oder ungesättigter, verzweigter oder unverzweigter Alkylrest mit 1 bis 4 Kohlenstoffatomen, welcher mindestens eine Hydroxylgruppe enthalten kann, oder
- ein gesättigter oder ungesättigter, verzweigter oder unverzweigter oder ein cyclischer gesättigter oder ungesättigter Alkylrest mit 6 bis 30 Kohlenstoffatomen, welcher mindestens eine Hydroxylgruppe enthalten kann, oder
- einen Aryl oder Alkylarylrest, beispielsweise Phenyl oder Benzyl, oder
- (- X - R8), mit der Maßgabe, dass höchstens 2 der Reste R5, R6 oder R7 für (- X - R8) stehen können, wobei
X die folgende Bedeutung hat:
- -(CH2)n- mit n = 1 bis 20, vorzugsweise n = 1 bis 10 und besonders bevorzugt n = 1 - 5, oder
- -(CH₂-CHR9-O)ₙ- mit n = 1 bis 200, vorzugsweise 1 bis 100, mehr bevorzugt 1 bis 50, und besonders bevorzugt 1 bis 20, sowie mit R9 in der Bedeutung von Wasserstoff, Methyl oder Ethyl, oder
- eine Hydroxyalkylengruppe mit ein bis vier Kohlenstoffatomen, welche verzweigt oder unverzweigt sein kann, und welche mindestens eine und höchstens 3 Hydroxylgruppen enthält, und wobei
R8 die folgende Bedeutung hat:
- R10-O-CO-, worin R10 einen gesättigten oder ungesättigten, verzweigten oder unverzweigten oder einen cyclischen, gesättigten oder ungesättigten Alkylrest mit 6 bis 30 Kohlenstoffatomen ist, welcher mindestens eine Hydroxylgruppe enthalten kann, und welcher gegebenenfalls weiterhin mit 1 bis 100 Ethylenoxideinheiten und/oder 1 bis 100 Propylenoxideinheiten oxethyliert sein kann, oder
- R11-CO-, worin R11 einen gesättigten oder ungesättigten, verzweigten oder unverzweigten oder einen cyclischen, gesättigten oder ungesättigten Alkylrest mit 6 bis 30 Kohlenstoffatomen ist, welcher mindestens eine Hydroxylgruppe enthalten kann, und welcher gegebenenfalls weiterhin mit 1 bis 100 Ethylenoxideinheiten und/oder 1 bis 100 Propylenoxideinheiten oxethyliert sein kann, und
in der A für ein physiologisch verträgliches organisches oder anorganisches Anion steht, bevorzugt steht einer der Reste R5, R6 oder R7 für die Gruppe (- X - R8), R8 steht für einen nicht ethoxylierten Fettsäurerest, wie für einen Palmitin-, Stearin-, Arachin- oder einen Behensäurerest, insbesondere einen Stearinsäurerest, und A steht für ein Halogenidion, ein Sulfation der allgemeinen Formel RSO₃⁻, worin R die Bedeutung von gesättigtem oder ungesättigten Alkylresten mit 1 bis 4 Kohlenstoffatomen hat, oder für einen anionischen Rest einer organischen Säuren wie Maleinsäure, Fumarsäure, Oxalsäure, Weinsäure, Citronensäure, Milchsäure oder Essigsäure, insbesondere für ein Chloridion oder für ein Methosulfation.

Die für die erfindungsgemäßen Mittel geeigneten Esterquats sind vorzugsweise ausgewählt aus mindestens einem der unter den Handelsbezeichnungen Rewoquat^{®}, Stepantex^{®}, Dehyquart^{®}, Armocare^{®} und Akypoquat^{®} vertriebenen Produkte. Spezifische Beispiele erfindungsgemäß besonders geeigneter Esterquats sind die Produkte Armocare^{®} VGH-70, Dehyquart^{®} F-75, Dehyquart^{®} C-4046, Dehyquart^{®} L80, Dehyquart^{®} F-30, Dehyquart^{®} AU-35, Rewoquat^{®} WE18, Rewoquat^{®} WE38 DPG, Stepantex^{®} VS 90 und Akypoquat^{®} 131.

Besonders bevorzugt sind erfindungsgemäße Mittel, die als Esterquat b) mindestens eine der unter den INCI-Bezeichnungen Distearoylethyl Hydroxyethylmonium Methosulfate und Distearoylethyl Hydroxyethylmonium Chloride bekannten Verbindungen enthalten.

Insbesondere bevorzugt ist Distearoylethyl Hydroxyethylmonium Methosulfate, das in den erfindungsgemäßen Mitteln in einer bevorzugten Menge von 0,1 bis 10 Gew.-%, mehr bevorzugt von 0,5 bis 8 Gew.-%, besonders bevorzugt von 0,75 bis 6 Gew.-% und insbesondere von 1 bis 5 Gew.-% enthalten sein kann, wobei sich die Mengenangaben auf das Gesamtgewicht des erfindungsgemäßen Mittels beziehen.

Das oder die Esterquats kann (können) den erfindungsgemäßen Mitteln sowohl einzeln, als auch als Mischung mit anderen Pflegewirkstoffen hinzugefügt werden.

Aufgrund der besseren Handhabbarkeit und Verarbeitbarkeit kann es von Vorteil sein, wenn das oder die Esterquats - insbesondere Distearoylethyl Hydroxyethylmonium Methosulfate - den erfindungsgemäßen Mitteln als Wirkstoffmischung zugegeben wird (werden). Ein besonders geeignetes Beispiel für eine solche Wirkstoffmischung ist beispielsweise unter der Handelsbezeichnung Dehyquart® F 75 von der Firma BASF erhältlich (Distearoylethyl Hydroxyethylmonium Methosulfate und Cetearyl Alcohol).

Die Abscheidung der Pflegewirkstoffe auf den Haaren ist ein besonders wichtiger Aspekt der Erfindung. Es wurde gefunden, dass die Abscheidung der Pflegewirkstoffe (a) und b)) besonders zufrieden stellend ist, wenn die erfindungsgemäßen Mittel als dritte Komponente c) ein quaternisiertes Hydroxyethylcellulosepolymer enthalten, welches kationische Trimethylammonium - und Dimethyldodecylammoniumgruppen als Substituenten aufweist.

Erfindungsgemäß besonders geeignete Komponenten c) sind bevorzugt ausgewählt aus Polymeren, die eine Gruppierung der folgenden Formel enthalten:

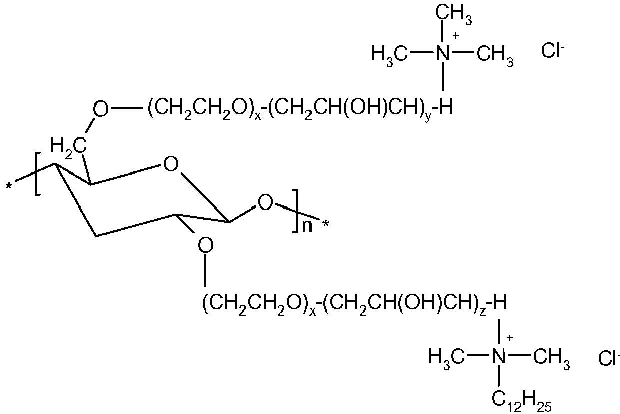

Weiterhin besonders geeignete Polymere c) weisen bevorzugt einen kationischen Substitutionsgrad von 0,15 bis 0,25 und einen hydrophoben Substitutionsgrad (HS) von HS ≤ 0,01 auf. Solche Polymere sind bekannt unter der INCI-Bezeichnung Polyquaternium-67 und werden im Handel beispielsweise von der Firma Amerchol unter den Bezeichnungen Polymer^{®} SL oder Polymer^{®} SK angeboten.

Insbesondere bevorzugte erfindungsgemäße Mittel enthalten ein unter der INCI-Bezeichnung Polyquaternium-67 bekanntes quaternisiertes Hydroxyethylcellulosepolymer c).

Polyquaternium-67 kann in den erfindungsgemäßen Mitteln, bezogen auf deren Gesamtgewicht - bevorzugt in einer Menge von 0,01 bis 5 Gew.-%, mehr bevorzugt von 0,05 bis 3 Gew.-%, besonders bevorzugt von 0,75 bis 2 Gew.-% und insbesondere von 0,1 bis 1 Gew.% enthalten sein.

In einer ersten besonders bevorzugten Ausführungsform enthalten erfindungsgemäße Mittel - bezogen auf ihr Gesamtgewicht -
a) 0,01 bis 10 Gew.-%, bevorzugt 0,05 bis 8 Gew.-% mindestens einer Verbindung der allgemeinen Formel (I), 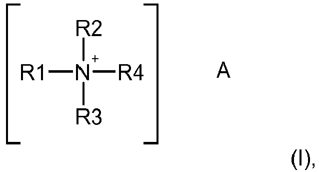 in der
   höchstens drei Reste R1 bis R4 unanhängig voneinander für eine gesättigte oder ungesättigte, verzweigte oder unverzweigte Alkylgruppe mit 1 bis 4 C-Atomen stehen, mindestens ein Rest R1 bis R4 für eine gesättigte oder ungesättigte, verzweigte oder unverzweigte Alkylkette mit 8 bis 30 C-Atomen steht, und A ein physiologisch verträgliches organisches oder anorganisches Anion bedeutet,
b) 0,01 bis 10 Gew.-%, bevorzugt 0,05 bis 8 Gew.-% mindestens eines Esterquats und
c) 0,01 bis 5 Gew.-%, bevorzugt 0,02 bis 4 Gew.-% mindestens eines quaternisierten Hydroxyethylcellulosepolymeren, welches kationische Trimethylammonium- und Dimethyldodecylammoniumgruppen als Substituenten aufweist.

Innerhalb dieser ersten besonders bevorzugten Ausführungsform ist es besonders bevorzugt, wenn die erfindunsgemäßen Mittel (bezogen auf ihr Gesamtgewicht)
a) 0,1 bis 7 Gew.-%, besonders bevorzugt 0,5 bis 6 Gew.-% und insbesondere 1 bis 5 Gew.-% mindestens einer Verbindung der zuvor genannten Formel (I), in der
   - drei Reste R1 bis R4 für eine Methylgruppe stehen,
   - ein Rest R1 bis R4 für eine Cetyl-, Palmityl-, Stearyl-, Arachidyl- oder eine Behenylgruppe steht, und
   - A für ein Chlorid oder ein Methosulfation steht,
b) 0,1 bis 7 Gew.-%, besonders bevorzugt 0,5 bis 6 Gew.-% und insbesondere 1 bis 5 Gew.-% mindestens eines der unter den INCI-Bezeichnungen Distearoylethyl Hydroxyethylmonium Methosulfate und Distearoylethyl Hydroxyethylmonium Chloride bekannten Esterquats, und
c) 0,03 bis 3 Gew.-%, besonders bevorzugt 0,04 bis 2 Gew.-% und insbesondere 0,05 bis 1 Gew.-% mindestens eines quaternisierten Hydroxyethylcellulosepolymeren enthalten, welches die folgende Gruppierung enthält: 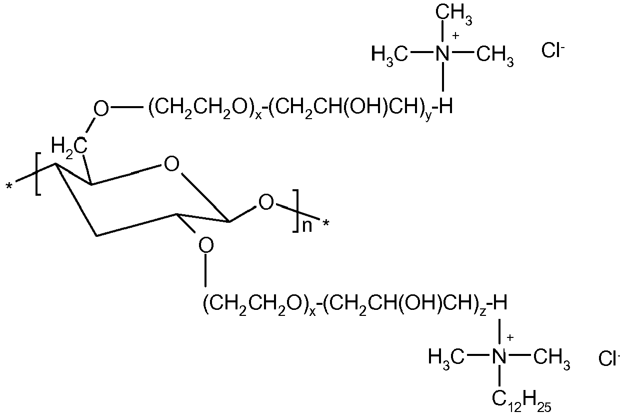
Innerhalb dieser ersten besonders bevorzugten Ausführungsform ist es insbesondere bevorzugt, wenn die erfindunsgemäßen Mittel (bezogen auf ihr Gesamtgewicht)
a) 0,1 bis 10 Gew.-%, mehr bevorzugt von 0,5 bis 8 Gew.-%, besonders bevorzugt von 0,75 bis 6 Gew.-% und insbesondere von 1 bis 5 Gew.-% eine der unter der INCI-Bezeichnung Behentrimethylammoniumchlorid bekannte Verbindung nach Formel (I),
b) 0,1 bis 10 Gew.-%, mehr bevorzugt von 0,5 bis 8 Gew.-%, besonders bevorzugt von 0,75 bis 6 Gew.-% und insbesondere von 1 bis 5 Gew.-% eines unter der INCI-Bezeichnung Distearoylethyl Hydroxyethylmonium Methosulfate bekannten Esterquats, und
c) 0,01 bis 5 Gew.-%, mehr bevorzugt von 0,05 bis 3 Gew.-%, besonders bevorzugt von 0,75 bis 2 Gew.-% und insbesondere von 0,1 bis 1 Gew.% eines unter der INCI-Bezeichnung Polyquaternium-67 bekannten kationischen Polymeren enthalten.

Es wurde gefunden, dass durch die Anwesenheit des speziellen kationischen Polymeren c) in den erfindungsgemäßen Mitteln nicht nur die Pflegekomponenten a) und b), sondern auch weitere (fakultativ in dem Mittel enthaltene) Pflegekomponenten, wie beispielsweise Vitamine und/oder Proteinhydrolysate, besser auf den Haaren abgeschieden werden können.

In einer zweiten besonders bevorzugten Ausführungsform enthalten erfindungsgemäße Mittel daher (zusätzlich zu den erfindungswesentlichen Komponenten a), b) und c)) bevorzugt mindestens einen Stoff aus der Gruppe der Vitamine, Provitamine und Vitaminvorstufen und/oder mindestens einen Stoff aus der Gruppe der Proteinhydrolysate.

Geeignete Proteinhydrolysate sind Proteinhydrolysate tierischer, pflanzlicher, mariner oder bakterieller Herkunft. Die Proteinhydrolysate können sowohl durch saure, basische, sauer - enzymatische, basisch - enzymatische als auch ausschließlich durch enzymatische Hydrolyse gewonnen werden. Besonders bevorzugt sind Proteinhydrolysate, welche durch enzymatische Hydrolyse gewonnen wurden. Bevorzugte Proteinhydrolysate weisen keinerlei enzymatische Aktivität mehr auf. Weiterhin bevorzugte Proteinhydrolysate weisen ein Molekulargewicht zwischen 500 Da und 5.000.000 Da auf, bevorzugt 1000 Da bis 2.000.000 Da, mehr bevorzugt 10.000 Da bis 1.000.000 Da, besonders bevorzugt 15.000 Da bis 500.000 Da und höchst bevorzugt 15.000 Da bis 100.000 Da.

Beispiele für bevorzugte Proteinhydrolysate sind Proteinhydrolysate auf der Basis von Keratin, Collagen, Gelatine, Albumin, Eiprotein, Lupinenprotein, Weizenprotein, Kartoffelprotein, Rapsprotein, Sojaprotein sowie Milchproteinen.

Besonders bevorzugte Proteinhydrolysate sind Proteinhydrolysate auf der Basis von Eiprotein, Keratin, Collagen, Gelatine, Sojaprotein, Albumin und Milch.

Insbesondere bevorzugt sind Proteinhydrolysate auf der Basis von Keratin.

Geeignete Keratinhydrolysate, die in den erfindungsgemäßen Mitteln vorzugsweise eingesetzt werden können, sind beispielsweise unter den INCI-Bezeichnungen Hydrolyzed Keratin und Cocodimonium Hydroxypropyl Hydrolyzed Keratin im Handel erhältlich (z.B. die Handelsprodukte Nutrilan^{®} Keratin W, Promois^{®} WK, Crotein^{®} K, ProSina^{®}, Promois^{®} WK HCAQ, Croquat^{®} WKP PE).

Die Proteinhydrolysate können in den erfindungsgemäßen Mitteln bevorzugt in einer Menge von 0,001 bis 7,5 Gew. %, mehr bevorzugt von 0,005 bis 5 Gew. %, besonders bevorzugt von 0,01 bis 2 Gew. % und höchst bevorzugt von 0,025 bis 1 Gew. % enthalten sein, wobei sich die Mengenangaben auf das Gesamtgewicht des jeweiligen Haarbehandlungsmittels beziehen. Besonders bevorzugt enthalten erfindungsgemäße Mittel mindestens eines der unter den INCI-Bezeichnungen Hydrolyzed Keratin und Cocodimonium Hydroxypropyl Hydrolyzed Keratin bekannten Keratinhydrolysate in einer bevorzugten Menge von 0,001 bis 5 Gew. %, mehr bevorzugt von 0,005 bis 3 Gew. %, besonders bevorzugt von 0,01 bis 2 Gew. % und höchst bevorzugt von 0,025 bis 0,5 Gew. %, wobei sich die Mengenangaben auf das Gesamtgewicht des jeweiligen Haarbehandlungsmittels beziehen.

Geeignete Vitamine, Pro-Vitamine und Vitaminvorstufen sind bevorzugt Vitamine, Pro-Vitamine und Vitaminvorstufen, die den Gruppen A, B, C, E, F und H zugeordnet werden.

*Vitamin A*: Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen beispielsweise Vitamin A-Säure und deren Ester, Vitamin A-Aldehyd und Vitamin A-Alkohol sowie dessen Ester wie das Palmitat und das Acetat in Betracht. Die erfindungsgemäßen Mittel enthalten die Vitamin A-Komponente bevorzugt in Mengen von 0,05-1 Gew.-%, bezogen auf die gesamte Zubereitung.

*Vitamin B*: Zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Bevorzugt ist das Nicotinsäureamid, das in den erfindungsgemäßen Haarbehandlungsmitteln bevorzugt in Mengen von 0,05 bis 1 Gew.-%, bezogen auf das gesamte Mittel, enthalten sein kann
- Vitamin B₅ (Pantothensäure, Panthenol und Pantolacton). Im Rahmen dieser Gruppe wird bevorzugt das Panthenol und/oder Pantolacton in den erfindungsgemäßen Mitteln eingesetzt. Einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie kationische Panthenolderivate. Die genannten Verbindungen des Vitamin B₅-Typs sind in den erfindungsgemäßen Mitteln bevorzugt in Mengen von 0,05 - 10 Gew.-%, bezogen auf das gesamte Mittel, enthalten. Mengen von 0,1 - 5 Gew.-% sind besonders bevorzugt.
- Vitamin B₆ (Pyridoxin sowie Pyridoxamin und Pyridoxal).

*Vitamin C* (Ascorbinsäure): Vitamin C wird in den erfindungsgemäßen Mitteln bevorzugt in Mengen von 0,1 bis 3 Gew.-%, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

*Vitamin E* (Tocopherole, insbesondere α-Tocopherol): Tocopherol und seine Derivate, worunter insbesondere die Ester wie das Acetat, das Nicotinat, das Phosphat und das Succinat fallen, sind in den erfindungsgemäßen Mitteln bevorzugt in Mengen von 0,05-1 Gew.-%, bezogen auf das gesamte Mittel, enthalten.

*Vitamin F*: Unter dem Begriff "Vitamin F" werden üblicherweise essentielle Fettsäuren, insbesondere Linolsäure, Linolensäure und Arachidonsäure, verstanden.

*Vitamin H:* Als Vitamin H wird die Verbindung (3aS,4S, 6aR)-2-Oxohexahydrothienol[3,4-d]-imidazol-4-valeriansäure bezeichnet, für die sich aber inzwischen der Trivialname Biotin durchgesetzt hat. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.

Erfindungsgemäß bevorzugte Mittel enthalten mindestens ein Vitamin, Provitamin oder eine Vitaminvorstufe aus den zuvor genannten Gruppen A, B, E und H.

Besonders bevorzugte erfindungsgemäße Mittel enthalten mindestens ein Vitamin, Provitamin oder eine Vitaminvorstufe, ausgewählt aus Niacinamid, Panthenol, Pantolacton, Tocopherolacetat und/oder Biotin in einer bevorzugten Menge von 0,001 bis 2 Gew.-%, mehr bevorzugt von 0,005 bis 1 Gew.-% und insbesondere von 0,01 bis 0,75 Gew.-%, bezogen auf das Gesamtgewicht des jeweiligen erfindungsgemäßen Mittels.

In einer dritten besonders bevorzugten Ausführungsform enthalten erfindungsgemäße Mittel (zusätzlich zu den Komponenten a), b) und c)), bezogen auf ihr Gesamtgewicht,
- 0,001 bis 7,5 Gew. %, mehr bevorzugt 0,005 bis 5 Gew. %, besonders bevorzugt 0,01 bis 2 Gew. % und höchst bevorzugt 0,025 bis 1 Gew. % mindestens eines Proteinhydrolysats und
- 0,001 bis 2 Gew.-%, mehr bevorzugt 0,005 bis 1 Gew.-% und insbesondere 0,01 bis 0,75 Gew.-% mindestens eines Vitamins, eines Vitaminderivats und/oder einer Vitaminvorstufe.

Innerhalb dieser Ausführungsform ist es besonders bevorzugt, wenn erfindungsgemäße Mittel, bezogen auf ihr Gesamtgewicht,
- 0,001 bis 7,5 Gew. %, mehr bevorzugt 0,005 bis 5 Gew. %, besonders bevorzugt 0,01 bis 2 Gew. % und höchst bevorzugt 0,025 bis 1 Gew. % mindestens eines Keratinhydrolysats, und
- 0,001 bis 2 Gew.-%, mehr bevorzugt 0,005 bis 1 Gew.-% und insbesondere 0,01 bis 0,75 Gew.-% mindestens eines Vitamins, eines Vitaminderivats und/oder einer Vitaminvorstufe aus den zuvor genannten Gruppen A, B, E und H enthalten.

Innerhalb dieser Ausführungsform ist es insbesondere bevorzugt, wenn erfindungsgemäße Mittel, bezogen auf ihr Gesamtgewicht,
- 0,001 bis 5 Gew. %, mehr bevorzugt 0,005 bis 3 Gew. %, besonders bevorzugt 0,01 bis 2 Gew. % und höchst bevorzugt 0,025 bis 0,5 Gew. % mindestens eines der unter den INCI-Bezeichnungen Hydrolyzed Keratin und Cocodimonium Hydroxypropyl Hydrolyzed Keratin bekannten Keratinhydrolysate, und
- 0,001 bis 2 Gew.-%, mehr bevorzugt 0,005 bis 1 Gew.-% und insbesondere 0,01 bis 0,75 Gew.-% Niacinamid, Panthenol, Pantolacton, Tocopherolacetat und/oder Biotin enthalten.

In einer weiteren Ausführungsform ist es bevorzugt, wenn die erfindungsgemäßen Mittel zusätzlich mindestens einen weiteren pflegenden Wirkstoff enthalten.

Der (die) weitere(n) pflegende(n) Wirkstoff(e) kann (können) bevorzugt ausgewählt sein aus natürlichen, synthetischen und/oder mineralischen Öl-, Fett- und/oder Wachskomponenten.

Geeignete Öl-, Fett- und/oder Wachskomponenten können in den erfindungsgemäßen Mitteln bevorzugt in Mengen von 0,05 bis 20 Gew.-%, besonders bevorzugt von 0,1 bis 15 Gew.-% und insbesondere von 0,5 bis 10 Gew.-% (bezogen auf das Gesamtgewicht des erfindungsgemäßen Mittels) enthalten sein.

Als natürliche (pflanzliche) Öle werden üblicherweise Triglyceride und Mischungen von Triglyceriden eingesetzt. Bevorzugte natürliche Öle sind Kokosnussöl, (süßes) Mandelöl, Walnussöl, Pfirsichkernöl, Aprikosenkernöl, Avocadoöl, Teebaumöl (Tea Tree Oil), Sojaöl, Sesamöl, Sonnenblumenöl, Tsubakiöl, Nachtkerzenöl, Reiskleieöl, Palmkernöl, Mangokernöl, Wiesenschaumkrautöl, Distelöl, Macadamianussöl, Traubenkernöl, Amaranthsamenöl, Arganöl, Bambusöl, Olivenöl, Weizenkeimöl, Kürbiskernöl, Malvenöl, Haselnussöl, Safloröl, Canolaöl, Sasanquaöl, Jojobaöl, Rambutanöl, Kakaoabutter und Shea-Butter.

Als mineralische Öle kommen insbesondere Mineralöle, Paraffin- und Isoparaffinöle sowie synthetische Kohlenwasserstoffe zum Einsatz. Ein Beispiel für einen einsetzbaren Kohlenwasserstoff ist beispielsweise das als Handelsprodukt erhältliche 1,3-Di-(2-ethylhexyl)-cyclohexan (Cetiol^{®} S).

Als synthetische Öle können Dialkylether, beispielsweise Di-n-octylether, Fettsäuren, Fettalkohole sowie natürliche und synthetische Wachse eingesetzt werden, welche sowohl in fester Form als auch flüssig in wässriger Dispersion vorliegen können.

Beispiele für geeignete Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen.

Bevorzugt sind üblicherweise die Fettsäureschnitte, welche aus Cocosöl oder Palmöl erhältlich sind; insbesondere bevorzugt ist in der Regel der Einsatz von Stearinsäure.

Geeignete Fettalkohole sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole. Als natürliche oder synthetische Wachse können eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP.

Als synthetische Öle kommen weiterhin Silikonverbindungen in Betracht.

Silikone bewirken auf dem Haar ausgezeichnete konditionierende Eigenschaften. Insbesondere bewirken sie eine bessere Kämmbarkeit der Haare in nassem und trockenem Zustand und wirken sich in vielen Fällen positiv auf den Haargriff und die Weichheit der Haare aus.

Es ist daher erstrebenswert, in den kosmetischen Haarbehandlungsmitteln Silikone einzusetzen. Geeignete Silikone können ausgewählt sein unter:
(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, die ausgewählt sind unter:
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
(iii) linearen Polysiloxan(A)- Polyoxyalkylen(B)- Blockcopoylmeren vom Typ (A-B)ₙ mit n > 3;
(iv) gepfropften Siliconpolymeren mit nicht siliconhaltigem, organischen Grundgerüst, die aus einer organischen Hauptkette bestehen, welche aus organischen Monomeren gebildet wird, die kein Silicon enthalten, auf die in der Kette sowie gegebenenfalls an mindestens einem Kettenende mindestens ein Polysiloxanmakromer gepfropft wurde;
(v) gepfropften Siliconpolymeren mit Polysiloxan- Grundgerüst, auf das nicht siliconhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silicon enthält;
(vi) oder deren Gemischen.

Weitere geeignete Fettstoffe sind beispielsweise
- Esteröle. Unter Esterölen sind zu verstehen die Ester von C₆ - C₃₀ - Fettsäuren mit C₂ - C₃₀ - Fettalkoholen. Bevorzugt sind beispielsweise Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®}868), Cetyloleat, Glycerintricaprylat, Kokosfettalkohol-caprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen,
- Glycerincarbonat oder Dicaprylylcarbonat (Cetiol^{®} CC),
- ethoxylierte oder nicht ethoxylierte Mono,- Di- und Trifettsäureester von gesättigten und/oder ungesättigten linearen und/oder verzweigten Fettsäuren mit Glycerin, wie beispielsweise Monomuls^{®} 90-018, Monomuls^{®} 90-L12, Cetiol^{®} HE oder Cutina^{®} MD.

Neben den zuvor genannten zwingenden und den bevorzugten fakultativen Komponenten können die erfindungsgemäßen Mittel weitere Wirkstoffe enthalten, die ihnen vorteilhafte Eingeschaften verleihen, beispielsweise weitere quaternäre Ammoniumverbindungen, die sich von den quaternären Ammoniumverbindungen a), b), c) unterscheiden:

Geeignete weiter quaternäre Ammoniumverbindungen können beispielsweise ausgewählt sein aus der Gruppe
- der quarternären Imidazoline gemäß Formel (II), 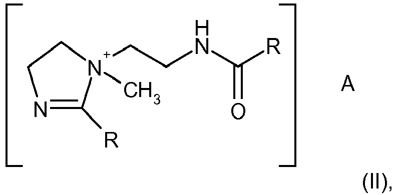 in welcher die Reste R unabhängig voneinander jeweils für einen gesättigten oder ungesättigten, linearen oder verzweigten Kohlenwasserstoffrest mit einer Kettenlänge von 8 bis 30 Kohlenstoffatomen und A für ein physiologisch verträgliches Anion steht, und/oder
- der Amine und/oder der kationisierten Amine und/oder
- der Poly(methacryloyloxyethyltrimethylammoniumverbindungen) und/oder
- der quaternisierten Cellulose-Derivate, insbesondere Polyquaternium-10, und/oder
- der kationischen Alkylpolyglycoside und/oder
- kationiserter Honig und/oder
- der kationischen Guar-Derivate und/oder
- Chitosan und/oder
- der polymeren Dimethyldiallylammoniumsalze und deren Copolymeren mit Estern und Amiden von Acrylsäure und Methacrylsäure und/oder
- der Copolymere des Vinylpyrrolidons mit quaternierten Derivaten des Dialkylaminoalkylacrylats und -methacrylats und/oder
- Vinylpyrrolidon-Vinylimidazoliummethochlorid-Copolymeren und/oder
- quaternierten Polyvinylalkohols und/oder
- Polyquaternium 2 und/oder Polyquaternium-7 und/oder Polyquaternium-16 und/oder Polyquaternium 17 und/oder Polyquaternium 18 und/oder Polyquaternium 24 und/oder Polyquaternium 27.

Bevorzugte quartäre Imidazolinverbindungen der Formel (II) enthalten für R jeweils den gleichen Kohlenwasserstoffrest. Die Kettenlänge der Reste R beträgt bevorzugt 12 bis 21 C-atome.

A steht für ein Anion wie zuvor beschrieben. Besonders bevorzugte Beispiele für zuvor genannte Imidazolinverbindungen sind unter den INCI - Bezeichnungen Quaternium-27, Quaternium-72, Quaternium-83 und Quaternium-91 erhältlich. Höchst bevorzugt ist Quaternium-91.

Bevorzugte Amine und/oder kationisierte Amine, insbesondere Amidoamine und/oder kationisierte Amidoamine weisen bevorzugt die folgende Strukturformel auf:

R1 - NH - (CH₂)ₙ - N⁺R²R³R⁴ A (III),

worin R1 bevorzugt einen Acyl-oder Alkylrest mit 6 bis 30 C-Atomen bedeuten kann, welcher verzweigt oder unverzweigt, gesättigt oder ungesättigt sein kann, und wobei der Acylrest und/oder der Alkylrest mindestens eine OH-Gruppe enthalten kann, und
R2, R3 und R4 jeweils unabhängig voneinander
1) Wasserstoff oder
2) ein Alkylrest mit 1 bis 4 C-Atomen, welcher gleich oder verschieden, gesättigt oder ungesättigt sein kann, und
3) eine verzweigte oder unverzweigte Hydroxyalkylgruppe mit ein bis 4 Kohlenstoffatomen mit mindestens einer und höchstens drei Hydroxygruppen beispielsweise -CH₂OH -CH₂CH₂OH, -CHOHCHOH, -CH₂CHOHCH₃, -CH(CH₂OH)₂, -COH(CH₂OH)₂, -CH₂CHOHCH₂OH, -CH₂CH₂CH₂OH und Hydroxybutylreste, bedeuten kann, und
A ein Anion wie zuvor beschrieben und
n eine ganze Zahl zwischen 1 und 10 bedeuten kann.

Besonders bevorzugt sind Amidoamine und/oder ein quaternisierte Amidoamine, worin R1 einen verzweigten oder unverzweigten, gesättigten oder ungesättigten Acylrest mit 6 bis 30 C-Atomen bedeutet, welcher mindestens eine OH-Gruppe enthalten kann. Bevorzugt ist hierbei ein Fettsäurerest aus Ölen und Wachsen, insbesondere aus natürlichen Ölen und Wachsen. Als Beispiele hierfür kommen Lanolin, Bienen-oder Candellilawachse in Betracht.

Bevorzugt sind auch solche Amidoamine und/oder quaternisierte Amidoamine, in denen R2, R3 und/oder R4 in der Formel (V) einen Rest gemäß der allgemeinen Formel CH₂CH₂OR5 bedeutet, worin R5 die Bedeutung von Alkylresten mit 1 bis 4 Kohlenstoffatomen, Hydroxyethyl oder Wasserstoff haben kann. Die bevorzugte Größe von n in der allgemeinen Formel (Tkat3) ist eine ganze Zahl zwischen 2 und 5.

Die Alkylamidoamine können sowohl als solche vorliegen, als auch durch Protonierung in entsprechend saurer Lösung in eine quaternäre Verbindung in der Zusammensetzung überführt werden. Erfindungsgemäß bevorzugt sind die kationischen Alkylamidoamine.

Beispiele für derartige Handelsprodukte sind Witcamine^{®} 100, Incromine^{®} BB, Mackine^{®} 401und andere Mackine^{®} -Typen, Adogen^{®} S18V, und als permanent kationische Aminoamine: Rewoquat^{®} RTM 50, Empigen^{®} CSC, Swanol^{®} Lanoquat DES-50, Rewoquat^{®} UTM 50, Schercoquat^{®} BAS, Lexquat^{®} AMG-BEO oder Incroquat^{®} Behenyl HE.

Die zuvor genannten - von a) und b) verschiedenen - kationischen Tenside können einzeln oder in beliebigen Kombinationen miteinander verwendet werden, wobei bevorzugt Mengen zwischen 0,01 bis 10 Gew.%, mehr bevorzugt von 0,01 bis 7,5 Gew.% und ganz besonders bevorzugt von 0,1 bis 5,0 Gew.% in den erfindungsgemäßen Mitteln verwendet werden. Die Mengenangaben beziehen sich auf das Gesamtgewicht der erfindungsgemäßen Haarbehandlungsmittel.

Bevorzugte - von c) verschiedene - kationische Polymere können ausgewählt sein aus: Homopolymeren der allgemeinen Formel -{CH₂-[CR¹COO-(CH₂)ₘN⁺R²R³R⁴]}ₙ X⁻,
in der R¹= -H oder -CH₃ ist, R², R³ und R⁴ unabhängig voneinander ausgewählt sind aus C1-4-Alkyl-, -Alkenyl- oder-Hydroxyalkylgruppen, m = 1, 2, 3 oder 4, n eine natürliche Zahl und
X⁻ ein physiologisch verträgliches organisches oder anorganisches Anion ist. Im Rahmen dieser Polymere sind diejenigen bevorzugt, für die mindestens eine der folgenden Bedingungen gilt: R¹ steht für eine Methylgruppe, R², R³ und R⁴ stehen für Methylgruppen, m hat den Wert 2.

Als physiologisch verträgliches Gegenionen X⁻ kommen beispielsweise Halogenidionen, Sulfationen, Phosphationen, Methosulfationen sowie organische Ionen wie Lactat-, Citrat-, Tartrat - und Acetationen in Betracht. Bevorzugt sind Methosulfate und Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Solche Produkte sind beispielsweise unter den Bezeichnungen Rheocare^{®} CTH (Cosmetic Rheologies) und Synthalen^{®} CR (3V Sigma) im Handel erhältlich.

Das Homopolymer wird bevorzugt in Form einer nichtwäßrigen Polymerdispersion eingesetzt. Solche Polymerdispersionen sind unter den Bezeichnungen Salcare^{®} SC 95 und Salcare^{®} SC 96 im Handel erhältlich.

Geeignete kationische Polymere, die von natürlichen Polymeren abgeleitet sind, sind kationische Derivate von Polysacchariden, beispielsweise kationische Derivate von Cellulose, Stärke oder Guar. Geeignet sind weiterhin Chitosan und Chitosanderivate. Kationische Polysaccharide weisen üblicherweise die allgemeine Formel G-O-B-N+RₐR_{b}R_{c} A⁻ auf, in der G einen Anhydroglucoserest bedeutet, beispielsweise Stärke- oder Celluloseanhydroglucose;

B eine divalente Verbindungsgruppe bedeutet, beispielsweise Alkylen, Oxyalkylen, Polyoxyalkylen oder Hydroxyalkylen;

Rₐ, R_{b} und R_{c} unabhängig voneinander Alkyl, Aryl, Alkylaryl, Arylalkyl, Alkoxyalkyl oder Alkoxyaryl mit jeweils bis zu 18 C-Atomen bedeuten, wobei die Gesamtzahl der C-Atome in Rₐ, R_{b} und R_{c} vorzugsweise maximal 20 ist; und

A⁻ ein übliches Gegenanion, vorzugsweise Chlorid, bedeutet.

Kationische, also quaternisierte, Cellulosen sind mit unterschiedlichem Substitutionsgrad, kationischer Ladungsdichte, Stickstoffgehalt und Molekulargewichten auf dem Markt erhältlich. Geeignete kationische Cellulosen sind unter den Bezeichnungen Polymer JR^{®} 400 (Amerchol, INCI-Bezeichnung Polyquaternium-10) sowie Polymer Quatrisoft^{®} LM-200 (Amerchol, INCI-Bezeichnung Polyquaternium-24) bekannt. Weitere Handelsprodukte sind die Verbindungen Celquat^{®} H 100 und Celquat^{®} L 200. Schließlich liegt unter der Handelsbezeichnung Mirustyle^{®} CP der Fa. Croda mit Trimonium and Cocodimonium Hydroxyethylcellulose eine weitere derivatisierte Cellulose mit der INCI-Bezeichnung Polyquaternium-72 vor. Polyquaternium-72 kann sowohl in fester Form als auch bereits in wässriger Lösung vorgelöst verwendet werden.

Besonders bevorzugte kationische Cellulosen sind Polyquaternium-10, Polyquaternium-24, und Polyquaternium-72.

Geeignete kationische Guarderivate werden unter der Handelsbezeichnung Jaguar^{®} vertrieben und haben die INCI-Bezeichnung Guar Hydroxypropyltrimonium Chloride. Weiterhin sind besonders geeignete kationische Guarderivate auch von der Fa. Hercules unter der Bezeichnung N-Hance^{®} im Handel erhältlich. Weitere kationische Guarderivate werden von der Fa. Cognis unter der Bezeichnung Cosmedia^{®} vertrieben. Ein bevorzugtes kationisches Guarderivat ist das Handelsprodukt AquaCat^{®} der Fa. Hercules. Bei diesem Rohstoff handelt es sich um ein bereits vorgelöstes kationisches Guarderivat.

Ein geeignetes Chitosan wird beispielsweise von der Firma Kyowa Oil& Fat, Japan, unter dem Handelsnamen Flonac^{®} vertrieben. Ein bevorzugtes Chitosansalz ist Chitosoniumpyrrolidoncarboxylat, welches beispielsweise unter der Bezeichnung Kytamer^{®} PC von der Firma Amerchol, USA, vertrieben wird. Weitere Chitosanderivate sind unter den Handelsbezeichnungen Hydagen^{®} CMF, Hydagen^{®} HCMF und Chitolam^{®} NB/101 im Handel frei verfügbar.

Weitere bevorzugte kationische Polymere sind beispielsweise
- kationische Alkylpolyglycoside,
- kationisierter Honig, beispielsweise das Handelsprodukt Honeyquat^{®} 50,
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere mit der INCI - Bezeichnung Polyquaternium-6 und Polyquaternium-7,
- Vinylpyrrolidon-Vinylimidazoliummethochlorid-Copolymere, wie sie unter den Bezeichnungen Luviquat^{®} FC 370, FC 550 und der INCI-Bezeichnung Polyquaternium-16 sowie FC 905 und HM 552 angeboten werden,
- quaternisiertes Vinylpyrrolidon/Dimethylaminoethylmethacrylat, zum Beispiel Vinylpyrrolidon/Dimethylaminoethylmethacrylatmethosulfat Copolymer, das unter den Handelsbezeichnungen Gafquat^{®} 755 N und Gafquat^{®} 734 von der Firma Gaf Co., USA vertrieben wird und die INCI - Bezeichnung Polyquaternium-11,
- quaternierter Polyvinylalkohol,
- sowie die unter den Bezeichnungen Polyquaternium-2, Polyquaternium-7, Polyquaternium-16, Polyquaternium-17, Polyquaternium- 18 Polyquaternium-24 und Polyquaternium-27 bekannten Polymeren mit quartären Stickstoffatomen in der Polymerhauptkette.

Die zuvor genannten - von c) verschiedenen - kationischen Polymere können einzeln oder in beliebigen Kombinationen miteinander verwendet werden, wobei die erfindungsgemäßen Mittel bevorzugt kationische Polymer-Mengen zwischen 0,01 bis 10 Gew.%, bevorzugt zwischen 0,01 bis 7,5 Gew.% und ganz besonders bevorzugt zwischen 0,1 bis 5,0 Gew.% enthalten (wobei sich die Mengenangaben auf das Gesamtgewicht der Haarbehandlungsmittel beziehen).

Weitere Inhaltsstoffe der Mittel gemäß Anspruch 1 können alle weiteren in kosmetischen Mitteln üblichen Inhaltsstoffe sein. Der Fachmann wird diese je nach dem Zweck des Mittels gezielt auswählen.

Im Folgenden werden einige ganz besonders bevorzugte weitere Inhaltsstoffe beispielhaft beschrieben.

Die erste bevorzugte Gruppe weiterer Inhaltsstoffe sind Pflanzenextrakte.

Bevorzugt sind vor allem die Extrakte aus Grünem Tee, Eichenrinde, Brennessel, Hamamelis, Hopfen, Henna, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Baldrian, Wiesenschaumkraut, Quendel, Schafgarbe, Thymian, Melisse, Hauhechel, Huflattich, Eibisch, Meristem, Ginseng, Kaffee, Kakao, Moringa, Ingwerwurzel und ayurvedische Pflanzenextrakte wie beispielsweise Aegle Marmelos (Bilwa), Cyperus Rotundus (Nagar Motha), Emblica Officinalis (Amalki), Morida Citrifolia (Ashyuka), Tinospora Cordifolia (Guduchi), Santalum album, (Chandana), Crocus Sativus (Kumkuma), Cinnamonum Zeylanicum und Nelumbo Nucifera (Kamala), Süßgräser wie Weizen, Gerste, Roggen, Hafer, Dinkel, Mais, die verschiedenen Sorten der Hirse (Rispenhirse, Fingerhirse, Kolbenhirse als Beispiele), Zuckerrohr, Weidelgras, Wiesenfuchsschwanz, Glatthafer, Straußgras, Wiesenschwingel, Pfeifengras, Bambus, Baumwollgras, Lampenputzergräser, Andropogonodeae (Imperata Cylindrica auch Flammengras oder Cogon Gras genannt), Büffelgras, Schlickgräser, Hundszahngräser, Liebesgräser, Cymbopogon (Zitronengras), Oryzeae (Reis), Zizania (Wildreis), Strandhafer, Staudenhafer, Honiggräser, Zittergräser, Rispengräser, Quecken und Echinacea, insbesondere Echinacea angustifolia DC, Echinacea paradoxa (Norton), Echinacea simulata, E. atrorubens, E. tennesiensis, Echinacea strigosa (Mc Gregor), Echinacea laevigata, Echinacea purpurea (L.) Moench und Echinacea pallida (Nutt), aller Arten von Wein sowie Perikarp von Litchie chinensis bevorzugt.

Die Pflanzenextrakte können sowohl in reiner als auch in verdünnter Form eingesetzt werden. Bezogen auf den Gehalt des jeweiligen Pflanzenextraktes werden Pflanzenextrakte in solchen Mengen verwendet, dass in den erfindungsgemäßen Zusammensetzungen Wirkstoffgehalte des jeweiligen Extraktes in einer Menge von 0,001 bis 7,5 Gew. %, bevorzugt 0,01 bis 5,0 Gew. % und besonders bevorzugt von 0,01 bis 3,0 Gew. % enthalten.

Eine zweite bevorzugte Gruppe weiterer Inhaltsstoffe in den erfindungsgemäßen kosmetischen Zusammensetzungen sind die im folgenden genannten Betaine: Carnitin, Carnitintartrat, Carnitin Magnesiumcitrat, Acetylcarnitin, Betalaine, 1,1-Dimethyl-Prolin, Cholin, Cholinchlorid, Cholinbitartrat, Cholindihydrogencitrat und die in der Literatur als Betain bezeichnete Verbindung N,N,N-trimethylglycin.

Bevorzugt werden Carnitin, Histidin, Cholin sowie Betain verwendet. Besonders bevorzugt ist L-Carnitintartrat.

Die zuvor genannten Betaine werden in den erfindungsgemäßen Mitteln - bezogen auf deren Gesamtgewicht - bevorzugt in Mengen von 0,00001 bis 10,0 Gew.-%, vorzugsweise von 0,0001 bis 5,0 Gew.-% und insbesondere von 0,001 bis 3 Gew.-% eingesetzt.

Ein dritter bevorzugter weiterer Inhaltsstoff ist Taurin und/oder ein Derivat des Taurins. Unter Taurin wird ausschließlich 2-Aminoethansulfonsäure und unter einem Derivat werden die explizit genannten Derivate des Taurins verstanden. Unter den Derivaten des Taurins werden N-Monomethyltaurin, N, N-Dimethyltaurin, Taurinlysylat, Taurintartrat, Taurinornithat, Lysyltaurin und Ornithyltaurin verstanden. Weitere geeignete Taurinderivate sind die Taurocholsäure und Hypotaurin.

Bevorzugt enthalten erfindungsgemäße Mittel - bezogen auf ihr Gewicht - 0,0001 bis 10,0 Gew.-%, vorzugsweise 0,0005 bis 5,0 Gew.-%, besonders bevorzugt 0,001 bis 2,0 Gew.-% und insbesondere 0,001 bis 1,0 Gew.-% Taurin und/oder ein zuvor genanntes Taurinderivat.

Eine vierte bevorzugte Gruppe weiterer Inhaltsstoffe in den erfindungsgemäßen Zusammensetzungen sind Biochinone. Unter geeigneten Biochinonen sind ein oder mehrere Ubichinon(e) und/oder Plastochinon(e) zu verstehen. Bevorzugte Ubichinone weisen die folgende Formel auf:

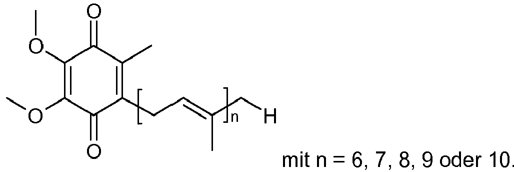

Das Coenzym Q-10 ist besonders bevorzugt.

Die erfindungsgemäßen Zusammensetzungen können - bezogen auf ihr Gesamtgewicht - bevorzugt 0,0001 bis 5 Gew.-%, mehr bevorzugt 0,0005 bis 3 Gew.-% und insbesondere 0,001 bis 1 Gew.-% der zuvor genannten Biochinone enthalten.

Eine fünfte bevorzugte Gruppe weiterer Inhaltsstoffe in den erfindungsgemäßen Zusammensetzungen Purin und/oder Purinderivate.

Bevorzugte erfindungsgemäße Mittel enthalten - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Purin und/oder Purinderivate.

Unter Purin, den Purinen und den Purinderivaten sind Purin, Adenin, Guanin, Harnsäure, Hypoxanthin, 6-Purinthiol, 6-Thioguanin, Xanthin, Coffein, Theobromin oder Theophyllin zu verstehen.

Insbesondere bevorzugt sind Coffein, Theobromin und/oder Theophyllin.

Ferner können die erfindungsgemäßen Mittel weitere Wirk-, Hilfs- und Zusatzstoffe in einer Menge bis maximal 5 Gew.-% (bezogen auf das Gesamtgewicht der erfindungsgemäßen Mittel) enthalten, wie beispielsweise
- UV - Lichtschutzfilter mit einer Wirkung im Bereich des UV-A, UV-B und UV-C Lichtes sowohl als wasserlösliche als auch öllösliche Filter,
- weitere kationische, amphotere, anionische oder nichtionische Polymere auf synthetischer Basis,
- Farbstoffvorprodukte vom Kuppler- und Entwicklertyp,
- Direktziehende Farbstoffe,
- Strukturanten wie Maleinsäure und Milchsäure,
- Quellmittel wie Harnstoff, Allantoin, Carbonate oder Hydantoin,
- Dimethylisosorbid und Cyclodextrine,
- Farbstoffe zum Anfärben des Mittels,
- Antischuppenwirkstoffe wie Piroctone Olamine, Zink Omadine und Climbazol,
- Komplexbildner wie EDTA, NTA, β-Alanindiessigsäure und Phosphonsäuren,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wasserstoffperoxid und andere Oxidationsmittel,
- Treibmittel wie Propan-Butan-Gemische, N₂O Dimethylether, CO₂ und Luft,
- Antioxidantien,
- Parfümöle, Duftstoffe und Riechstoffe.

Bezüglich weiterer fakultativer Komponenten sowie die eingesetzten Mengen dieser Komponenten wird ausdrücklich auf die dem Fachmann bekannten einschlägigen Handbücher verwiesen.

Die erfindungsgemäßen Mittel weisen hervorragende Eigenschaften auf: Bei regelmäßiger Anwendung der Mittel auf den Haaren werden hohe Mengen an Pflegewirkstoffen auf den Haaren abgeschieden, wodurch der Haargriff und das optische Erscheinungsbild der behandelten Haare signifikant verbessert werden kann.

Insbesondere zuvor geschädigte, beispielsweise stumpfe, glanzlose, brüchige und/oder gesplisste Haare, können durch die regelmäßige Verwendung der erfindungsgemäßen Mittel wieder in Ihrer Struktur gekräftigt werden, so dass sich das ästhetische Gesamtbild der Haare (Glätte, Glanz) insgesamt verbessern lässt.

Ein zweiter Gegenstand der Erfindung ist daher Verwendung des erfindungsgemäßen Mittels zur
- Kräftigung der Haarstruktur,
- Verbesserung des Haargriffs, insbesondere des Haargriffs trockener Haare, und
- Verbesserung des ästhetischen Gesamtbildes der Haare.

### Beispiele:

### 1) Beispielrezepturen

Die folgende Tabelle enthält Beispiele für erfindungsgemäße Haarkonditioniermittel (1,2: Treatment; 3,4: Conditioner).

Die Mengenangaben in der Tabelle beziehen sich auf Gew.-%.

| | **1** | **2** | **3** | **4** |
|---|---|---|---|---|
| **Cetearyl Alcohol** | 4,5 | 6 | 4 | 3 |
| **Dicaprylyl Carbonate** | 0,5 | 1 | 0,5 | 0,5 |
| **Glycol Distearate** | 1,5 | 2 | 0,5 | 0,5 |
| **Distearoyl Hydroxyethylmonium Methosulfate** | 2 | 2 | 2 | 2 |
| **Behentrimonium Chloride** | 2 | 2,5 | 3 | 2 |
| **Polyquatemium-67** | 0,1 | 0,15 | 0,2 | 0,25 |
| **Dimethicone** | 1 | 1,5 | 1 | 1,5 |
| **D-Panthenol** | 0,2 | 0,2 | 0,2 | 0,2 |
| **Glycerin** | 0,7 | 0,7 | 0,2 | 0,2 |
| **Parfum** | 0,6 | 0,7 | | |
| **Isopropylmyristat** | | 0,2 | | 1 |
| **Quaternium-87** | | | | 1,5 |
| **Behenoyl PG-Trimonium Chloride** | | | | 0,2 |
| **Hydrolyzed Keratin** | 0,1 | 0,2 | 0,15 | |
| **Cocodimonium Hydroxypropyl Hydrolyzed Keratin** | 0,2 | 0,1 | | 0,4 |
| **Niacinamide** | 0,1 | 0,2 | | |
| **Säuerungs-, Konservierungs-, Verdickungsmittel** | q.s. | q.s. | q.s. | q.s. |
| **Wasser** | ad 100 | ad 100 | ad 100 | ad 100 |

### 2) Wirkung der Haarkonditionierenden Mittel

Die Zusammensetzung des Beispiels 1 wurde einmal wie in Tabelle 1 hergestellt (I), einmal ohne einen Gehalt an kationischen Polymeren (II) und einmal mit 0,1 Gew.-% des im Stand der Technik bekannten kationischen Pflegepolymeren Polyquaternium-37 (III).

Anschließend wurden die Zusammensetzungen (I), (II) und (III) in zwei Halbseitentests ((I) gegen (II) und (I) gegen (III)) von Fachleuten beurteilt.
a) (I) gegen (II): Es wurde gefunden, dass die erfindungsgemäße Zusammensetzung auf nassen und trockenen Haaren einen signifikant verbesserten Griff sowie eine nachweislich verbesserte Entwirrbarkeit und Kämmbarkeit aufweist. Ebenso weisen trockene Haare, die mit der erfindungsgemäßen Zusammensetzung (I) behandelt wurden, mehr Glanz auf.
b) (I) gegen (III): Auch gegenüber Zusammensetzung (III), die ein kationisches Abscheidungspolymer (Polyquaternium-37) enthält, weisen die erfindungsgemäßen Zusammensetzungen signifikant verbesserte Pflegeergebnisse hinsichtlich Entwirrbarkeit, Kämmbarkeit, Haargriff, Griff der Haarspitzen, Body und Glanz auf. Gleichzeitig konnte beobachtet werden, dass die Langzeitwirkung der erfindungsgemäßen Zusammensetzung besser war als die Langzeitwirkung der Zusammensetzung (III), enthaltend das kationische Polymer Polyquaternium-37.

## Patentansprüche

1. Mittel zur Konditionierung von Keratinfasern, insbesondere von Haaren, enthaltend in einem kosmetischen Träger
a) mindestens eine Verbindung der nachfolgenden Formel (I) 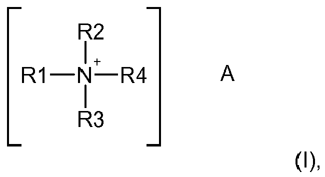 in der
höchstens drei Reste R1 bis R4 unanhängig voneinander für eine gesättigte oder ungesättigte, verzweigte oder unverzweigte Alkylgruppe mit 1 bis 4 C-Atomen stehen, mindestens ein Rest R1 bis R4 für eine gesättigte oder ungesättigte, verzweigte oder unverzweigte Alkylkette mit 8 bis 30 C-Atomen steht, und A ein physiologisch verträgliches organisches oder anorganisches Anion bedeutet,
b) mindestens ein Esterquat und
c)mindestens ein quaternisiertes Hydroxyethylcellulosepolymer, das kationische Trimethylammonium- und Dimethyldodecylammoniumgruppen als Substituenten enthält.

2. Mittel nach Anspruch 1, **dadurch gekennzeichnet, dass** es - jeweils bezogen auf sein Gesamtgewicht -
- 0,01 bis 10 Gew.-%, bevorzugt 0,05 bis 8 Gew.-%, mehr bevorzugt 0,1 bis 7 Gew.-%, besonders bevorzugt 0,5 bis 6 Gew.-% und insbesondere 1 bis 5 Gew.-% der Komponente a),
- 0,01 bis 10 Gew.-%, bevorzugt 0,05 bis 8 Gew.-%, mehr bevorzugt 0,1 bis 7 Gew.-%, besonders bevorzugt 0,5 bis 6 Gew.-% und insbesondere 1 bis 5 Gew.-% der Komponente b), und
- 0,01 bis 5 Gew.-%, bevorzugt 0,02 bis 4 Gew.-%, mehr bevorzugt 0,03 bis 3 Gew.-%, besonders bevorzugt 0,04 bis 2 Gew.-% und insbesondere 0,05 bis 1 Gew.-% der Komponente c) enthält.

3. Mittel nach einem der Ansprüche 1 oder 2, **dadurch gekennzeichnet, dass** die Komponente a) für Verbindungen gemäß Formel (I) steht, in denen zwei oder drei Reste R1 bis R4 für eine Methyl- oder eine Ethylgruppe stehen, ein oder zwei Rest(e) R1 bis R4 für eine gesättigte oder ungesättigte, verzweigte oder unverzweigte Alkylkette mit 14 bis 26 C-Atomen steht(en) und worin A für ein Halogenidion, ein Sulfation der allgemeinen Formel RSO₃⁻, worin R die Bedeutung von gesättigtem oder ungesättigtem Alkylresten mit 1 bis 4 Kohlenstoffatomen hat, oder für einen anionischen Rest einer organischen Säuren wie Maleinsäure, Fumarsäure, Oxalsäure, Weinsäure, Citronensäure, Milchsäure oder Essigsäure, steht.

4. Mittel nach Anspruch 3, **dadurch gekennzeichnet, dass** die Komponente a) für Verbindungen gemäß Formel (I) steht, in denen drei Reste R1 bis R4 für eine Methylgruppe stehen, ein Rest R1 bis R4 für eine Cetyl-, Palmityl-, Stearyl-, Arachidyl- oder eine Behenylgruppe steht, und worin A für ein Chlorid oder ein Methosulfation steht, bevorzugt steht a) für Cetyltrimethylammoniumchlorid, Cetyltrimethylammoniummethosulfat, Behentrimethylammoniumchlorid oder Behentrimethylammoniummethosulfat und besonders bevorzugt für Behentrimethylammoniumchlorid.

5. Mittel nach einem der Ansprüche 1 bis 4, **dadurch gekennzeichnet, dass** das Esterquat b) ausgewählt ist aus Verbindungen der nachfolgenden Formel (II) 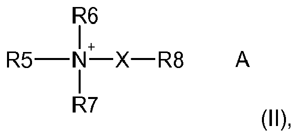 in der
die Reste R5, R6 und R7 jeweils unabhängig voneinander gleich oder verschieden sein können und die folgende Bedeutung haben:
- ein gesättigter oder ungesättigter, verzweigter oder unverzweigter Alkylrest mit 1 bis 4 Kohlenstoffatomen, welcher mindestens eine Hydroxylgruppe enthalten kann, oder
- ein gesättigter oder ungesättigter, verzweigter oder unverzweigter oder ein cyclischer gesättigter oder ungesättigter Alkylrest mit 6 bis 30 Kohlenstoffatomen, welcher mindestens eine Hydroxylgruppe enthalten kann, oder
- einen Aryl oder Alkylarylrest, beispielsweise Phenyl oder Benzyl, oder
- (- X - R8), mit der Maßgabe, dass höchstens 2 der Reste R5, R6 oder R7 für (- X - R8) stehen können, wobei
X die folgende Bedeutung hat:
- -(CH2)n- mit n = 1 bis 20, vorzugsweise n = 1 bis 10 und besonders bevorzugt n = 1 - 5, oder
- -(CH₂-CHR9-O)ₙ- mit n = 1 bis 200, vorzugsweise 1 bis 100, mehr bevorzugt 1 bis 50, und besonders bevorzugt 1 bis 20, sowie mit R9 in der Bedeutung von Wasserstoff, Methyl oder Ethyl, oder
- eine Hydroxyalkylengruppe mit ein bis vier Kohlenstoffatomen, welche verzweigt oder unverzweigt sein kann, und welche mindestens eine und höchstens 3 Hydroxylgruppen enthält, und wobei
R8 die folgende Bedeutung hat:
- R10-O-CO-, worin R10 einen gesättigten oder ungesättigten, verzweigten oder unverzweigten oder einen cyclischen, gesättigten oder ungesättigten Alkylrest mit 6 bis 30 Kohlenstoffatomen ist, welcher mindestens eine Hydroxylgruppe enthalten kann, und welcher gegebenenfalls weiterhin mit 1 bis 100 Ethylenoxideinheiten und/oder 1 bis 100 Propylenoxideinheiten oxethyliert sein kann, oder
- R11-CO-, worin R11 einen gesättigten oder ungesättigten, verzweigten oder unverzweigten oder einen cyclischen, gesättigten oder ungesättigten Alkylrest mit 6 bis 30 Kohlenstoffatomen ist, welcher mindestens eine Hydroxylgruppe enthalten kann, und welcher gegebenenfalls weiterhin mit 1 bis 100 Ethylenoxideinheiten und/oder 1 bis 100 Propylenoxideinheiten oxethyliert sein kann, und
in der A für ein physiologisch verträgliches organisches oder anorganisches Anion steht, bevorzugt steht einer der Reste R5, R6 oder R7 für die Gruppe (- X - R8), R8 steht für einen nicht ethoxylierten Fettsäurerest, wie für einen Palmitin-, Stearin-, Arachin- oder einen Behensäurerest, insbesondere einen Stearinsäurerest, und A steht für ein Halogenidion, ein Sulfation der allgemeinen Formel RSO₃⁻, worin R die Bedeutung von gesättigtem oder ungesättigten Alkylresten mit 1 bis 4 Kohlenstoffatomen hat, oder für einen anionischen Rest einer organischen Säuren wie Maleinsäure, Fumarsäure, Oxalsäure, Weinsäure, Citronensäure, Milchsäure oder Essigsäure, insbesondere für ein Chloridion oder für ein Methosulfation.

6. Mittel nach Anspruch 5, **dadurch gekennzeichnet, dass** es als Esterquat mindestens eine der unter den INCI-Bezeichnungen Distearoylethyl Hydroxyethylmonium Chloride und Distearoylethyl Hydroxyethylmonium Methosulfate bekannten Verbindungen enthält.

7. Mittel nach einem der Ansprüche 1 bis 5, **dadurch gekennzeichnet, dass** es als quaternisiertes Hydroxyethylcellulosepolymer c) mindestens eines der unter der INCI-Bezeichnung Polyquaternium-67 bekannten kationischen Polymere enthält.

8. Mittel nach einem der Ansprüche 1 bis 7, **dadurch gekennzeichnet, dass** es zusätzlich mindestens einen Stoff aus der Gruppe der Vitamine, Provitamine und Vitaminvorstufen und/oder mindestens einen Stoff aus der Gruppe der Proteinhydrolysate enthält.

9. Mittel nach einem der Ansprüche 1 bis 8, **dadurch gekennzeichnet, dass** es bezogen auf sein Gesamtgewicht - zusätzlich 0,005 bis 10 Gew.-%, bevorzugt 0,01 bis 5 Gew.-% und insbesondere 0,05 bis 3 Gew.-% mindestens einer Öl-, Wachs- und/oder Fettkomponente enthält.

10. Verwendung eines Mittels nach einem der Ansprüche 1 bis 9 zur
- Kräftigung der Haarstruktur,
- Verbesserung des Haargriffs, insbesondere des Haargriffs trockener Haare, und
- Verbesserung des ästhetischen Gesamtbildes der Haare.
